(19) Europäisches Patentamt
European Patent Office
Office européen des brevets

(11) Publication number: 0 035 270
A2

(12) # EUROPEAN PATENT APPLICATION

(21) Application number: 81101497.6

(22) Date of filing: 02.03.81

(51) Int. Cl.³: C 07 D 231/08, A 61 K 31/415

(30) Priority: 03.03.80 GB 8007201

(43) Date of publication of application: 09.09.81
Bulletin 81/36

(84) Designated Contracting States: BE CH DE FR GB LI LU NL SE

(71) Applicant: THE WELLCOME FOUNDATION LIMITED,
183-193 Euston Road, London NW1 2BP (GB)

(72) Inventor: Ingold, Kenneth James, 119 Temple Lane,
Durham North Carolina 27713 (US)
Inventor: Howard, James Lawrence, Route 1, Chapel Hill
North Carolina 27514 (US)
Inventor: McDermed, John Dale, 130 Larkspur Circle,
Durham North Carolina (US)

(74) Representative: Berg, Wilhelm, Dr. et al, Dr. Berg,
Dipl.-Ing. Stapf, Dipl.-Ing. Schwabe, Dr. Dr. Sandmair
Mauerkircherstrasse 45, D-8000 München 80 (DE)

(54) **Heterocyclic compounds, compositions thereof, their preparation and use.**

(57) Heterocyclic compounds, more specifically 1-substituted pyrazolidin-3-ones of the formula (I)

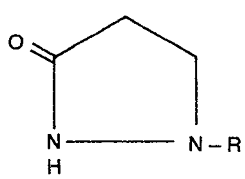

(I)

wherein R is a straight chain alkyl group of from 8 to 14 carbon atoms and acid addition salts thereof are disclosed, together with pharmaceutical formulations thereof. The compounds of formula (I) are useful in medicine, particularly as anorexigenic agents.

EP 0 035 270 A2

The present invention relates to heterocyclic compounds which are useful in medicine, to the preparation of such compounds, pharmaceutical formulations containing them, and their use in medicine.

It has been found that pyrazolidinones as further defined below in formula (I) and their corresponding acid addition salts, have anorectic properties and are useful in the control of appetite in mammals, including man.

The anorectic properties of the pyrazolidinones have been demonstrated by their ability to suppress the weight increase which usually occurs in normal rats, and in some instances to effect a decrease in the body weight of such rats.

A disadvantage of amphetamine and amphetamine-like anorectic agents currently in use is their stimulant effect on the central nervous system resulting in undesirable side effects such as nervousness and insomnia. In addition, some amphetamine-like compounds such as dexamphetamine and phenmetrazine have been found to cause euphoria and a consquent tendency to dependence on such drugs by the user. The pyrazolidinones of the present invention are structurally unrelated to amphetamine and have not shown a comparable stimulant action on the central nervous system. The anorectic effect of the pyrazolidinones is also more persistent and less susceptible to tolerance than that of fenfluoramic.

In formula (I):

wherein $R^1$ is straight alkyl having from 7 to 13 carbon atoms. Appropriate reactants and reaction conditions for reducing a compound of formula (II) will be known to those skilled in the art and include catalytic hydrogenation using for example Raney nickel or a noble metal catalyst such as platinum or palladium. If desired the hydrogen may be employed at a greater than atmospheric pressure to facilitate the reaction and the reaction is preferably carried out using an inert solvent. Suitable solvents include hydrocarbons such as hexane or toluene, and lower alkanols such as methanol or ethanol. Alternatively the compounds of formula (II) may be reduced using conventional reducing agents such as sodium cyanoborohydride or nascent hydrogen which may for example be obtained by reacting iron, zinc or tin in an acid such as hydrochloric acid or acetic acid, or sodium in ethanol.

The intermediates of formula (II) may readily be obtained by reacting 3-pyrazolidone, or preferably an acid addition salt thereof such as the hydrochloride, with an appropriate aldehyde of formula (III):

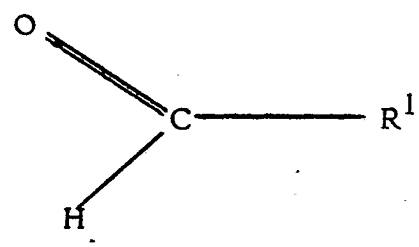

(III)

wherein $R^1$ has the same meaning as in formula (II). Although the aldehyde and 3-pyrazolidone may react directly, the reaction is facilitated by the presence of a dehydrating agent such as phosphorous pentoxide in ether or by the use of azeotropic distillation or molecular sieves.

It is not necessary to isolate the compound of formula (II) and conveniently this compound is prepared *in situ* and the reaction medium then used directly for reduction to provide the desired compound of formula (I).

wherein $R^1$ is straight alkyl having from 7 to 13 carbon atoms. Appropriate reactants and reaction conditions for reducing a compound of formula (II) will be known to those skilled in the art and include catalytic hydrogenation using for example Raney nickel or a noble metal catalyst such as platinum or palladium. If desired the hydrogen may be employed at a greater than atmospheric pressure to facilitate the reaction and the reaction is preferably carried out using an inert solvent. Suitable solvents include hydrocarbons such as hexane or toluene, and lower alkanols such as methanol or ethanol. Alternatively the compounds of formula (II) may be reduced using conventional reducing agents such as sodium cyanoborohydride or nascent hydrogen which may for example be obtained by reacting iron, zinc or tin in an acid such as hydrochloric acid or acetic acid, or sodium in ethanol.

The intermediates of formula (II) may readily be obtained by reacting 3-pyrazolidone, or preferably an acid addition salt thereof such as the hydrochloride, with an appropriate aldehyde of formula (III):

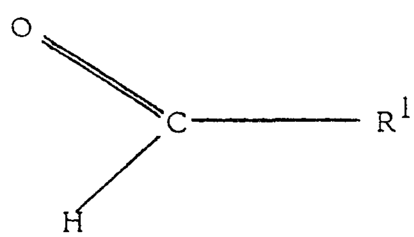

(III)

wherein $R^1$ has the same meaning as in formula (II). Although the aldehyde and 3-pyrazolidone may react directly, the reaction is facilitated by the presence of a dehydrating agent such as phosphorous pentoxide in ether or by the use of azeotropic distillation or molecular sieves.

It is not necessary to isolate the compound of formula (II) and conveniently this compound is prepared *in situ* and the reaction medium then used directly for reduction to provide the desired compound of formula (I).

The pyrazolidinones of formula (I) may also be prepared by alkylation of the pyrazolidone ring. That is, pyrazolidinone or an acid addition salt thereof is reacted with an alkylating agent which may be designated as a reactive ester derivative of an alcohol R.OH wherein R has the same meaning as in formula (I). Suitable reactive ester derivatives include halide (e.g. chloride, bromide or iodide) and sulphonate esters such as p-toluenesulphonate, methanesulphonate, benzenesulphonate and naphthalensulphonate esters. The reaction may be performed in the absence of a solvent but preferably in an inert solvent such as an alkanol (e.g. methanol), an aromatic hydrocarbon (e.g. benzene or toluene), an aliphatic ketone (e.g. acetone or methyl ethyl ketone), dioxan, tetrahydrofuran, dimethylsulphoxide, acetonitrile or dimethylformamide. Although the reaction may proceed without heating, elevated temperatures up to the reflux temperature of the reaction medium may be used and optionally under pressure.

A further means for preparing the desired compounds of formula (I) is formation of the pyrazolidinone ring, for example by cyclisation of a carboxylic acid of formula (IV):

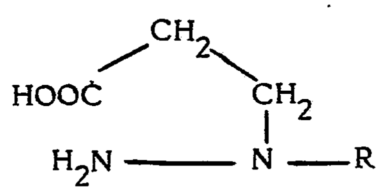

(IV)

wherein R is as defined in formula (I), or a reactive derivative thereof such as an acid halide, in particular the acid chloride or bromide, an acid anhydride, a lower alkyl ester or lower alkylthio ester wherein the alkyl group has 1 to 4 carbon atoms for example methyl or ethyl, or other activated ester such as an ester of N-hydroxysuccinamide or an aryl ester such as that with p-nitrophenol or thiophenol.

Suitable reaction conditions are similar to those used for the analogous formation of an amide and will be known to those skilled in the art. For example, the reaction may be effected under acidic or neutral conditions desirable with heating and in the presence of an inert solvent such as are mentioned above. The reaction may be promoted by use of a coupling agent such as dicyclohexylcarbodiimide.

Conveniently, the reaction conditions for the preparation of the compound of formula (IV) are chosen such that it cyclises spontaneously without prior isolation. For example, a compound of formula (IV) may be obtained by reducing a compound of formula (V):

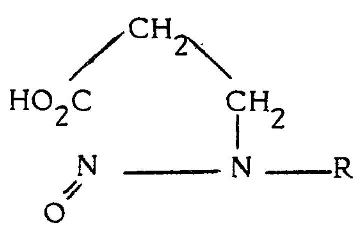

(V)

wherein R has the same meaning as in formula (I), or a corresponding reactive derivative thereof. Any reducing agent appropriate for this type of reaction may be used, for example those mentioned above for reducing a compound of formula (II), or acidic stannous chloride in a suitable solvent such as an alkanol e.g. ethanol, or aluminium analgam which may be employed in the presence of an alkanol or benzene/alkanol solvent. The reactants should of course be chosen to be compatible with the compound of formula (V), for example aluminium amalgam is in-appropriate for reducing the parent acid and should only be used for reducing a corresponding reactive derivative.

Intermediate compounds of formula (V) are conveniently prepared by reacting nitrous acid with a secondary amine of formula (VI):

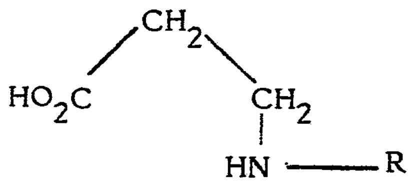

(VI)

wherein R is as defined in formula (II), or a reactive derivative thereof; the reaction being effected with cooling, at a temperature not exceeding 5°C.

The secondary amines of formula (VI) are readily obtained by reacting an appropriate acrylate ester with a primary amine of formula $RNH_2$.

Intermediates of formula (IV) may also be prepared by the reaction of an acrylate salt or ester with a compound of formula (VII):

$H_2H.NH.R$ (VII)

wherein R is as defined in formula (I). The reaction is preferably carried out in an alcoholic solution and at a temperature not exceeding 10°C.

As a further example, 3-pyrazolidinones of formula (I) may be prepared by hydrolysis of a corresponding 3-iminopyrazolidine or tautomeric 3-aminopyrazoline of formulae (VIIIA) and (VIIIB) respectively.

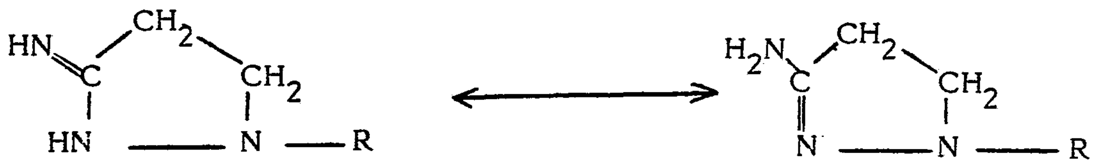

(VIIIA) (VIIIB)

wherein R is as defined in formula (I). The hydrolysis may be achieved by heating

in the presence of a dilute mineral acid or by steam distillation.

The compounds of formulae (VIII) may be prepared by methods analogous to those described above for the preparation of pyrazolidinones of formula (I) by reduction of a compound of formula (II) or by reaction of acrylonitrile with a compound of formula (VII).

A compound of formula (I) may be isolated as the base or corresponding acid addition salt thereof; and it will be appreciated that a base so obtained may be converted to an acid addition salt thereof, or an acid addition salt may be converted to the desired base or salt by conventional techniques.

The compounds of formula (I) may be used to control appetite or bodyweight in mammals such as mice, rats, dogs and cats, and more importantly man. They are of particular value in controlling and reducing obesity and, as such, they are useful in the prophylaxis of adverse conditions associated with obesity such as coronary artery disease, hypertension and maturity onset diabetes.

The effective anorectic dose in a particular situation will depend on the compound chosen, the recipient, the mode of administration and the severity of the condition to be treated, but will generally lie in the range if from 10 to 200 mg/kg bodyweight per day. A convenient intravenous dose is from 10 to 30 mg/kg, and an oral dose is preferably from 25 to 125 mg/kg bodyweight. Administration of the desired daily dose is preferably in three divided doses. For example, convenient forms of administration include tablets each containing from 100 to 500 mg of compound of formula (I).

For use in medicine the compounds of formula (I) may be administered as a pure chemical but are preferably presented with an acceptable carrier therefor as a pharmaceutical formulation. The carrier must of course be 'acceptable' in the sense of being compatible with the other ingredients of the formulation and not deleterious to the recipient of the formulation. The carrier may be a solid or a liquid or a mixture of solid and liquid substances, and is preferably formulated with a compound of formula (I) as a unit-dose formulation, for example a tablet, capsule or cachet for oral administration or a suppository for rectal administration. Other pharmaceutically active substances may also be present in formulations of the present invention, and the formulation may be formulated by any of the well-known techniques of pharmacy consisting basically of admixture of its components. Unit dose formulations, for oral, rectal or parenteral administration (vid. inf.) conveniently contain a compound of formula (I) in an amount in the range frim 10 mg to 500 mg.

For oral administration, fine powders or granules of the compounds may contain diluents and dispersing and surface active agents, and may be presented in a draught in water or in a syrup, in capsules or cachets in the dry state or in an aqueous or non-aqueous suspension, when a suspending agent may also be included; in tablets, preferably made from granules of the active ingredient with a diluent, by compression with binders and lubricants; or in a suspension in water or a syrup or an oil in a water/oil emulsion, when flavouring, preserving, suspending, thickening and emulsifying agents may also be included. The granules or the tablets may be coated, and the tablets may be scored.

For parenteral administration (by intramuscular or intraperitoneal injection), the compounds may be presented in unit dose or multi-dose containers in aqueous or non-aqueous injection solutions which may contain antioxidants, buffers, bacteriostats and solutes which render the compounds isotonic with the blood, or in aqueous or non-aqueous suspensions when suspending agents and thickening agents may also be included; extemporaneous injection solutions

and suspensions may be made from sterile powders, granules or tablets which may contain diluents, dispersing and surface active agents, binders and lubricants.

The following examples are provided to illustrate the invention but should not be construed as a limitation thereof.

EXAMPLE 1

Preparation of 1-(n-undecyl)-3-pyrazolidone hydrochloride

3-Pyrazolidone hydrochloride (12.12 g) was neutralised with sodium methylate and to the resulting solution of 3-pyrazolidone in methanol (150 ml) was added undecanal (25.5 g). The mixture, containing 1-(n-undecylidene)-3-pyrazolidone betaine was hydrogenated using hydrogen under 2 atmospheres of pressure in the presence if platinum (from 50 mg $PtO_2$) with agitation for 25 minutes. The catalyst was removed by filtration, the solvent removed by evaporation under reduced pressure and the residual oil extracted with ether/-chloroform (300 ml, 1:1). The solvent was evaporated from the extract, leaving an oil. Ethanolic hydrogenchloride was added to the oil, and then ether was added, precipitating a white solid. This was recrystallised from methanol/ether to give 1-(n-undecyl)-3-pyrazolidone hydrochloride m.p. 185-187°C.

EXAMPLE 2

By methods analogous to those described in Example 1 were prepared
1-(n-octylidene)-3-pyrazolidone betaine,
1-(n-nonylidene)-3-pyrazolidone betaine,
1-(n-decylidene)-3-pyrazolidone betaine, and
1-(n-tetradecylidene)-3-pyrazolidone betaine,
which were hydrogenated to provide the corresponding

1-(n-octyl)-3-pyrazolidone hydrochloride, m.p. 181-183°C

1-(n-nonyl)-3-pyrazolidone hydrochloride, m.p. 182.5-183.5°C

1-(n-tetradecyl)-3-pyrazolidone hydrochloride, m.p. 187-188°C.

1-(n-decyl)-3-pyrazolidone hydrochloride, m.p. 186-188°C.

EXAMPLE 3

Preparation of 1-(n-decyl)-3-pyrazolidone hydrochloride

3-Pyrazolidone hydrochloride (18.38 g) was neutralized with sodium methylate (8.10 g) in methanol (75 ml). The mixture was stirred for 5 minutes, diethyl ether (175 ml) added and the precipitated sodium chloride removed by filtration. The filtrate was evaporated to about 20 ml under reduced pressure at 40°C, and decanal (31.26 g) was added. Methanol was added to bring the volume of the mixture to 250 ml. The resulting solution, containing 1-(n-decylidene)-3-pyrazolidone betaine, was hydrogenated using hydrogen under 2-3 atmosphere of pressure in the presence of platinum (from 75 mg $PtO_2$) for 75 minutes. The catalyst was removed by filtration, and the solvent was removed by evaporation under reduced pressure. The residual oil was dissolved in diethyl ether, and the ethereal solution was dried over anhydrous sodium sulfate. Treatment with dry hydrogen chloride/ethanol precipitated 1-(n-decyl)-3-pyrazolidone hydrochloride. The precipitate was thoroughly extracted with boiling hexane and recrystallized from 2-propanol/diethyl ether to give pure 1-(n-decyl)-3-pyrazolidone hydrochloride, m.p. 186-188°C.

EXAMPLE 4

Pharmaceutical Formulations

In the following examples the term "Active Ingredient" represents any compound of formula (I) as the base; appropriate amendment to the stated

dose must be made if an acid addition salt is used.



| Tablet | Amount per tablet (mg) |
|---|---|
| Active Ingredient | 300.0 |
| Lactose | 98.0 |
| Corn Starch | 62.0 |
| Polyvinylpyrrolidone | 5.0 |
| Magnesium | 2.0 |

| Capsule | Amount per capsule (mg) |
|---|---|
| Active Ingredient | 300.0 |
| Lactose | 75.0 |
| Corn starch | 23.0 |
| Stearic acid | 2.0 |

| Ampoule | Amount per ampoule |
|---|---|
| Active Ingredient | 300.0 mg |
| Water for injection, q.s. | 1.0 ml |

| Suppository | Amount per suppository |
|---|---|
| Active Ingredient | 300.0 mg |
| Theobroma oil (cocoa butter), q.s. | 2.0 g |

## EXAMPLE 5

### Effect of Pyrazolidinones on bodyweight of Rats

The compound of formula (I) prepared by the methods described in Examples 1 and 2 were administered orally to rats at a dose corresponding to 0.22 m moles/kilogram bodyweight. Two days after administration the weight

of the treated rats was compared with control and the difference expressed as a percentage of the weight of the control animals.

| Compound (I) R = | % weight deviation from control |
|---|---|
| n-octyl | - 6.8 |
| n-nonyl | - 12.1 |
| n-decyl | -10.9 |
| n-undecyl | - 2.3 |

EXAMPLE 6

Effect of varying doses of 1-n-decyl pyrazolidin-3-one in rat Bodyweight

Male Sprague-Dawley rats were acclimatized to laboratory conditions for approximately four weeks prior to the experiment being carried out. The rats were maintained on a 12 hr/12 hr light dark cycle and had continuous access to food and water. Rats weighing approx. 575 grams were housed 4 to a cage and each rat treated daily with an oral injection of either saline vechicle or one of three doses of 1-n-decylpyrazolidinone dissolved in saline vehicle. The saline vehicle was prepared fresh each day. The results are shown in Table 1.

TABLE 1

| Treatment | Dose mg/kg p.o | Number of Rats treated | Percent Change from Initial Bodyweight (Mean ± S.E.M.) | |
|---|---|---|---|---|
| | | | Day 5 | Day 10 |
| Saline Vehicle (Control) | - | 8 | -1.3 ± 0.4 | +2.4 ± 1.1 |
| Compound (I) R= n-decyl | 10 | 8 | -2.7 ± 0.3 | -1.1 ± 0.4 |
| Compound (I) R=n-decyl | 15 | 8 | -3.3 ± 1.0 | -9.6 ± 1.7* |
| Compound (I) R=n-decyl | 20 | 7 | -3.8 ± 0.6* | -11.1 ± 1.8* |

* p .01 Compared to vehicle control group by Student's t-test.

EXAMPLE 7

Effect of 1-n-decyl pyrazolidin-3-one on Rat Bodyweight and Food Consumption

Male Sprague-Dawley rats were acclimatized as described in Example 6. The Rats (approx. 300 grams) were housed individually to present measurement of food consumption as well as bodyweight. Animals were treated once daily by oral comsumption as well as bodyweight. Animals were treated once daily by oral guage either with saline or with 1-n-decyl pyrazolidin-3-one (25 mg/kg) in saline vehicle. The effects on bodyweight and food consumption are shown in Tables 2 and 3 respectively. Measurement of bodyweight and food consumption was saturated during the recovery days, when treatment with drug has stopped.

TABLE 2

Present change for initial bodyweight (Mean ± S.E.M.)

| Treatment | Treatment Day | | | | | | Recovery Day | | |
|---|---|---|---|---|---|---|---|---|---|
| | 2 | 3 | 4 | 5 | 6 | 7 | 1 | 2 | 3 |
| Saline (N=6) | +2.7<br>±0.3 | +2.9<br>±0.6 | +4.1<br>±0.7 | +4.9<br>±0.5 | +3.9<br>±0.2 | +5.4<br>±1.1 | +5.2<br>±1.1 | +6.3<br>±1.1 | +6.9<br>±1.4 |
| Compound (I)<br>R=n-decyl<br>25 mg/kg p.o.<br>(N=8) | +0.3*<br>±0.7 | -1.8*<br>±0.8 | -2.4*<br>±0.7 | -4.6*<br>±1.3 | -5.0*<br>±1.3 | -9.5*<br>±1.8 | -13.4*<br>± 2.0 | -10.4*<br>± 1.9 | -5.9*<br>±1.6 |

* p .01 Compared to vehicle control group by Student's t test.

Table 3

Percent change in amount of food consumed (Mean ± S.E.M.)

| Treatment | Treatment Day | | | | | | Recovery Day | | |
|---|---|---|---|---|---|---|---|---|---|
| | 2 | 3 | 4 | 5 | 6 | 7 | 1 | 2 | 3 |
| Saline Vehicle (N=6) | +9<br>±6 | +6<br>±5 | +8<br>±6 | +2<br>±2 | +3<br>±2 | -10<br>±13 | +2<br>±8 | -4<br>±5 | +5<br>±7 |
| Compound (I) R=*n*-decyl 25 mg/kg p.o. (N=8) | -1<br>±6 | -15*<br>±4 | -17*<br>±4 | -45*<br>±12 | -39*<br>±9 | -69*<br>±13 | -80*<br>±6 | -8<br>±7 | +13<br>±8 |

* p 0.1 Compared to vehicle control group by Student's *t* test.

EXAMPLE 8

LD$_{50}$'s in mice

The LD$_{50}$'s of compounds of formula were determined by conventional techniques by i.p. administration to mice. The results are shown in Table 4.

TABLE 4

| 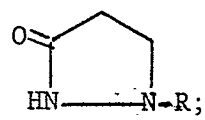 R= | LD$_{50}$ mg/kg |
|---|---|
| $CH_3(CH_2)_7$- | 250 |
| $CH_3(CH_2)_8$- | 210 |
| $CH_3(CH_2)_9$- | 500 |
| $CH_3(CH_2)_{10}$- | 350 |
| $CH_3(CH_2)_{13}$- | 350 |

We claim:-

1. Compounds of the formula (I)

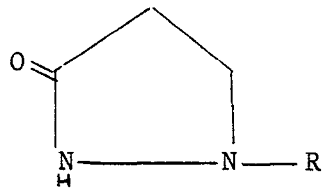

(I)

wherein R is a straight chain alkyl group having from 8 to 14 carbon atoms, and acid addition salts thereof.

2. A compound of formula (I) as claimed in claim 1 wherein R has 9 or 10 carbon atoms or an acid addition salt thereof.

3. 1-n-decylpyrazolidin-3-one or an acid addition salt thereof.

4. A pharmaceutical formulation comprising a compound of formula (I) as defined in claim 1 or a pharmaceutically acceptable salt thereof together with a pharmaceutically acceptable carrier therefor.

5. A pharmaceutical formulation as claimed in claim 5 in unit dosage form.

6. A method for the preparation of a compound of formula (I), as defined in claim 1 which method comprises:-

(a) reduction of a compound of formula (II)

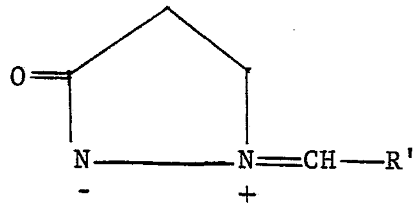

(II)

wherein R' is a straight chain alkyl group having from 7 to 13 carbon atoms; or

(b) cyclisation of a compound of formula (IV)

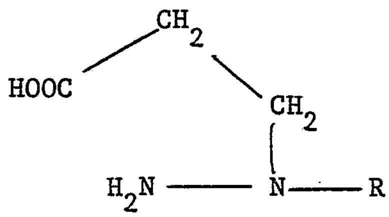

(IV)

wherein R is as defined in formula (I); or

(c) hydrolysis of a compound of formula (VIIIA) or a tautomer thereof

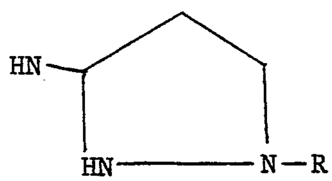

(VIIIA)

wherein R is as defined in formula (I).

7. A compound of formula (I) as defined in claim 1 or a pharmaceutically acceptable salt thereof for use in medicine.

8. A compound of formula (I) as defined in claim 1 or a pharmaceutically acceptable salt thereof for use as an anorexogenic agent or as an appetite suppressant.

9. A compound of formula (I) as claimed in either claim 7 or claim 8 wherein the compound is 1-n-decylpyrazolidin-3-one or a pharmaceutically acceptable acid addition salt thereof.